# EUROPEAN PATENT APPLICATION

(11) **EP 4 640 137 A1**
(43) Date of publication of application: **29.10.2025**
(21) Application number: 23907290.3
(22) Date of filing: 05.09.2023
(51) Int. Cl.: A61B 5/00, A61B 5/318, A61B 5/022, A61B 5/103

(54) **METHOD FOR MEASURING BIOMETRIC INFORMATION**

(30) Priority: 22.12.2022 KR 20220181687
(71) Applicant: VUNO INC., Seoul 06541 (KR)
(72) Inventor: BAE, Junwoo, Seoul 06541 (KR); LIM, Seokhun, Seoul 06541 (KR); JUNG, Kyunghoon, Seoul 06541 (KR)
(74) Representative: Petraz, Gilberto Luigi
(86) International application number: PCT/KR2023/013227
(87) International publication number: WO 2024/136002

(57) **Abstract**

Disclosed is a method performed by a measurement device for measuring biometric information according to one embodiment of the present disclosure. According to one embodiment of the present disclosure, the measurement device identifies a measurement attempt applied to the measurement device, performs both an operation of activating the measurement device and an operation of transmitting a wake-up signal to an external device, in response to the identified measurement attempt, and transmits measurement information to the external device.

## Description

### [Technical Field]

The present disclosure relates to a biometric information measurement method, and more particularly, to a method for identifying a measurement attempt by utilizing a measurement device, performing biometric information measurement, and transmitting measurement information to an external device by using a measurement device.

### [Background Art]

Biometric information means information that can be used as an indicator of human physical activity including biological electricity, bio magnetic, pressure, and sound. As a device for measuring the biometric information, a lot of hand-held devices which operate by linking with an external device such as a smartphone, etc., by a communication technology such as Bluetooth, etc. have been developed in recent years.

However, the biometric information measurement devices operating by linking with such external devices (e.g., smart devices, etc.) require user inputs, i.e., a measurement start input, a measurement end input, an analysis start input, etc. without exception. The biometric information should be measured periodically according to the condition and disease of the user, and manipulating the external device linked with the measurement device, preparing for the measurement, and obtaining a measurement result at each measurement causes a lot of inconvenience for the user.

Moreover, in an emergency situation, the need for the manipulation makes it difficult to quickly obtain the biometric information, and as a result, there is a possibility of making it impossible to quickly react to patients.

Therefore, there is a demand in the industry for measuring devices that can obtain the biometric information without the need to manipulate external devices separately.

Korean Registration Patent No. KR1995153B1 discloses "Method for Compensation of ECG Signal of Wearable Device Using Acceleration Sensor and ECG Measurement Wearable Device Adopting Same".

### [Disclosure]

### [Technical Problem]

The present disclosure has been made in an effort to utilize a measurement device which operates by linking with an external device, and measure biometric information quickly centered on the measurement device without a direct manipulation for the linked external device (e.g., a smart device, etc.), and provide a measurement result.

Meanwhile, a technical object to be achieved by the present disclosure is not limited to the above-mentioned technical object, and various technical objects can be included within the scope which is apparent to those skilled in the art from contents to be described below.

**In** order to implement the object described above, one embodiment of the present disclosure provides a method performed by a measurement device for measuring biometric information. The method may include: identifying a measurement attempt applied to the measurement device, performing both an operation of activating the measurement device and an operation of transmitting a wake-up signal to an external device, in response to the identified measurement attempt, and transmitting measurement information to the external device.

**In** an alternative embodiment, the transmitting of the measurement information to the external device may be performed while a measurement program for the measurement device is not performed in a foreground of the external device.

**In** an alternative embodiment, a signal received from the external device may be automatically generated based on an analysis of the measurement information without an input of a user into the external device.

In an alternative embodiment, the performing of both the operation of activating the measurement device and the operation of transmitting the wake-up signal to the external device, in response to the identified measurement attempt may further include generating an activation notification, and generating a measurement start notification based on identifying whether the measurement attempt meets a measurement start condition.

In an alternative embodiment, the method may further include performing at least one of an operation of deactivating the measurement device and an operation of transmitting an idle signal to the external device when the identified measurement attempt is stopped.

In an alternative embodiment, the method may further include performing an operation of deactivating the measurement device.

In an alternative embodiment, the method may further include terminating a measurement operation based on a signal received from the external device, and the terminating of the measurement operation based on the signal received from the external device may include performing at least one of the operation of terminating the measurement operation, an operation of transmitting a standby signal to the external device, and the operation of deactivating the measurement device when the signal is a measurement completion signal, or terminating the measurement operation when a predetermined time elapsed from a time when the signal is received.

In order to implement the object described above, another embodiment of the present disclosure provides a method performed by a computing device which operates by linking with a measurement device. The method may include: executing a program for the measurement device in a background when a wake-up signal is received from the measurement device; and analyzing measurement information received from the measurement device based on the program executed in the background.

In an alternative embodiment, the method may further include transmitting, when a unstable measurement state is identified through the analysis, feedback information for the unstable measurement state to the measurement device.

In an alternative embodiment, the method may further include outputting an analysis result by switching the program into a foreground state when the analysis of the measurement information is completed.

In an alternative embodiment, the unstable measurement state may be identified based on at least one of an intensity of the measurement information, an input time interval of the measurement information, and a length of the measurement information.

In order to implement the object described above, yet another embodiment of the present disclosure provides a computer program stored in a computer readable storage medium, which includes instructions which allow a measurement device to perform operations of measuring biometric information. The operations may include: an operation of identifying a measurement attempt applied to the measurement device, an operation of performing both an operation of activating the measurement device and an operation of transmitting a wake-up signal to an external device, in response to the identified measurement attempt, and an operation of transmitting measurement information to the external device.

In order to implement the object described above, still yet another embodiment of the present disclosure provides a measurement device for obtaining biometric information. The measurement device may include: at least one processor; a measurement unit for measuring biometric information; a communication unit for communicating with an external device, and an output unit providing a notification, and the processor may be configured to identify a measurement attempt applied to the measurement unit, perform both an operation of activating the measurement device and an operation of transmitting a wake-up signal to an external device, in response to the identified measurement attempt, and transmit measurement information to the external device.

### [Advantageous Effects]

According to one embodiment of the present disclosure, it is possible to utilize a measurement device which operates by linking with an external device, and measure biometric information quickly centered on the measurement device without a direct manipulation for the linked external device (e.g., a smart device, etc.), and provide a measurement result.

### [Description of Drawings]

FIG. 1 is a block diagram of a computing device including a measurement device for measuring biometric information according to one embodiment of the present disclosure.
FIG. 2 is a flowchart illustrating a process of measuring biometric information according to one embodiment of the present disclosure.
FIG. 3 is an exemplary diagram illustrating a 6-lead hand-held electrocardiogram measurement device among measurement devices according to one embodiment of the present disclosure.
FIG. 4 is an exemplary diagram illustrating a 2-lead hand-held electrocardiogram measurement device among the measurement devices according to one embodiment of the present disclosure.
FIG. 5 is an exemplary graph of an electrocardiogram measured based on an electrical signal received from an electrode according to one embodiment of the present disclosure.
FIG. 6 is an exemplary diagram illustrating a blood pressure measurement device among the measurement devices according to one embodiment of the present disclosure.
FIG. 7 is an exemplary diagram illustrating a weight measurement device among the measurement devices according to one embodiment of the present disclosure.
FIG. 8 is a simple and normal schematic view of an exemplary computing environment in which embodiments of the present disclosure may be implemented.

### [Mode for Invention]

The present disclosure relates to a method which quickly measures biometric information centered on a measurement device, and provides a measurement result, without separately manipulating a linked external device.

Various embodiments will now be described with reference to drawings. In this specification, various descriptions are presented to provide appreciation of the present disclosure. However, it is apparent that the embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

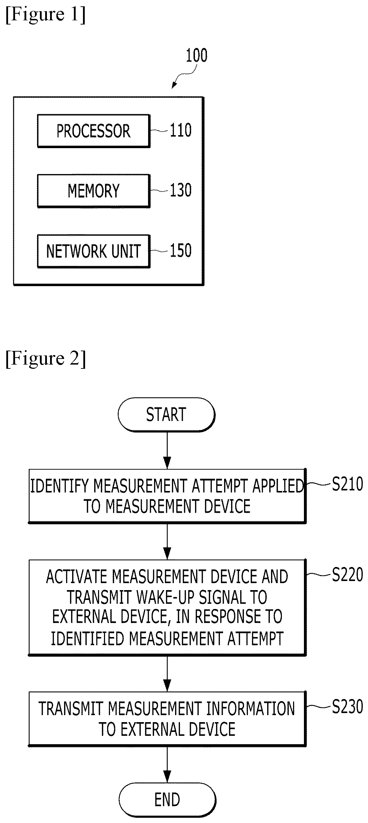

FIG. 1 is a block diagram of a computing device including a measurement device for measuring biometric information according to one embodiment of the present disclosure.

A configuration of the computing device 100 illustrated in FIG. 1 is only a simplified example. In one embodiment of the present disclosure, the computing device 100 may include other components for performing the computing environment of the computing device 100, and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like.

At least one of the CPU, GPGPU, and TPU of the processor 110 may process learning of a network function. For example, both the CPU and the GPGPU may process the learning of the network function and data classification using the network function. Further, in an exemplary embodiment of the present disclosure, processors of a plurality of computing devices may be used together to process the learning of the network function and the data classification using the network function. Further, the computer program executed in the computing device according to an exemplary embodiment of the present disclosure may be a CPU, GPGPU, or TPU executable program.

According to one embodiment of the present disclosure, the processor 110 included in the measurement device may identify a measurement attempt applied to the measurement device. A specific description regarding the processor 110 identifying the measurement attempt will be described below with reference to FIG. 2.

According to one embodiment of the present disclosure, the processor 110 included in the measurement device may perform both an operation of activating the measurement device in response to the identified measurement attempt, and an operation of transmitting a wake-up signal to the external device. Here, the wake-up signal may mean a signal that receives data transmitted by the measurement device, and changes the received data to a state in which the received data may be recorded. A specific description for the operation of activating the measurement device and transmitting the wake-up signal to the external device will be made below with reference to FIG. 2.

According to one embodiment of the present disclosure, the processor 110 included in the measurement device may transmit measurement information to the external device. The measurement device and the external device may include a communication module for transmitting and receiving data, and two devices may be linked through wired connection or wireless pairing. The wireless pairing may be performed by utilizing various technologies including Bluetooth and Wi-Fi technologies, etc.

According to one embodiment of the present disclosure, the processor 110 included in the measurement device may terminate a measurement operation under a predetermined condition. A specific method of terminating the measurement operation by the processor will be described below with reference to FIG. 2.

According to one embodiment of the present disclosure, the processor 110 included in the measurement device may perform both an operation of deactivating the measurement device by identifying a case where the measurement attempt is stopped without depending on a stop signal received from the external device, and an operation of transmitting an idle signal to the external device. For example, when the measurement device is a 6-lead hand-held measurement device for measuring an electrocardiogram, a part of a body needs to be in contact with each of three electrodes in order to measure the electrocardiogram. When the body is not in contact with some electrodes, a connection with the external device is broken, an acceleration of a predetermined speed or more is identified, or another abnormal situation occurs, the processor 110 may identify that the measurement attempt is stopped. At this time, the processor 110 of the hand-held electrocardiogram measurement device performs the operation of deactivating the measurement device and transmits the idle signal to the linked external device to be switched to the state to identify the measurement attempt again. An example of deactivating the measurement device and switching the external device to the idle state is not limited to the electrocardiogram measurement device, but other measurement devices for blood pressure, weight, body temperature, etc., may perform such an operation.

In the present disclosure, there is an effect in which the measurement device identifies the case where the measurement attempt is stopped to resolve a hassle that must stop measurement through an input of a user into the external device, and reduce a time for which biometric information is measured.

Further, the processor 110 included in the measurement device may perform an operation of transmitting the measurement information to the external device, and then deactivating the measurement device. For example, when the measurement device is the 6-lead hand-held measurement device for measuring the electrocardiogram, the processor 110 may perform an operation of obtaining electrocardiogram data as the measurement information, transmitting the obtained measurement information to the external device, and then immediately deactivating the measurement device. As another example, the processor 110 may perform an operation of transmitting the measurement information to the external device, and then deactivating the measurement device after a predetermined time (e.g., 3 seconds) elapsed.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to several embodiments of the present disclosure may use various wired communication systems, such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a local area network (LAN).

The network unit 150 presented in the present specification may use various wireless communication systems, such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

The network unit 150 according to an exemplary embodiment of the present disclosure may use an arbitrary type known wired/wireless communication systems.

The techniques described herein may be used in other networks in addition to those mentioned above.

As one embodiment of the present disclosure, a method performed by an external device (e.g., a smart device, etc.) which operates by linking with the measurement device is initiated. Here, the external device may be implemented in a form of including the computing device similarly to the measurement device described above. Further, the external device may include the processor, the memory, the network unit, etc.

Meanwhile, the method performed by the external device may be implemented as a form such as a program, an application, etc.

According to one embodiment of the present disclosure, when the external device receives a wake-up signal from the measurement device, the external device may execute a program for the measurement device in a background. Further, the external device may analyze the measurement information received from the measurement device based on the program executed in the background. Thereafter, according to a result of analyzing the measurement information, the external device may transmit a measurement end signal to the measurement device when a measurement completion condition is achieved. Additionally, when an unstable measurement state is identified according to the result of analyzing the measurement information, the external device may transmit feedback information for the unstable measurement state to the measurement device. At this time, the unstable measurement state may be identified based on at least one of an intensity of the measurement information, an input time interval of the measurement information, and a length of the measurement information.

FIG. 2 is a flowchart illustrating a process of measuring biometric information according to one embodiment of the present disclosure.

According to FIG. 2, the process of measuring the biometric information according to the present disclosure may include a step (S210) of identifying a measurement attempt applied to the measurement device, a step (S220) of activating the measurement device and transmitting a wake-up signal to the external device, in response to the identified measurement attempt, and a step (S230) of transmitting measurement information to the external device.

In step S210, the processor 110 included in the measurement device may identify the measurement attempt applied to the measurement device. In the present disclosure, the measurement attempt encompasses various attempts of manipulating, by a user, the measurement device in order to measure the biometric information. For example, the measurement device may include a gyroscope or an acceleration sensor, and a sensor may sense a change such as the user's moving or lifting the measurement device, and the processor 110 may identify a reaction of the sensor as the measurement attempt. As another example, the measurement device may include a contact sensor, and the sensor of the measurement device senses that a skin of the user is in contact with an electrode of the measurement device, so the processor 110 may identify the reaction of the sensor as the measurement attempt. However, the measurement attempt of the present disclosure is not limited to the method taken as an example.

In step S220, the processor 110 included in the measurement device may perform both an operation of activating the measurement device and an operation of transmitting the wake-up signal to the external device, in response to the identified measurement attempt. At this time, 'activation' may mean an operation of changing a state of a measurement equipment so as to receive the input of the user and record a bio-signal by the measurement device. Further, in step S220 in which the 'wake-up signal' allows the external device to deviate from the idle state, and transmitted by the measurement device, the processor 110 included in the measurement device may perform both an operation of activating the measurement device and an operation of transmitting the wake-up signal to the external device, in response to the identified measurement attempt. At this time, the 'activation' may mean an operation of changing a state of a measurement equipment so as to receive the input of the user and record a bio-signal by the measurement device. Further, the 'activation signal' may mean an operation of allowing the external device to deviate from the idle state, and changing the state of the external device so as to receive, record, and analyze the measurement information transmitted by the measurement device. The external device that receives the wake-up signal may receive, record, and analyze the measurement information transmitted from the measurement device without a separate user input.

Step S220 may include a step of generating an activation notification. The activation notification may mean an operation of notifying, to the user, that the measurement device is activated by starting of the measurement device, a contact of the electrode included in the measurement device, or movement of the hand-held measurement device, and biometric information of the user may be prepared to be received. Further in step S220, a measurement start notification may be generated based on identifying whether the measurement attempt satisfies a measurement condition. At this time, the measurement condition may be a condition for whether the input from the user is suitable for measuring the biometric information. For example, when the measurement device is a 6-lead electrocardiogram measurement device having three electrodes, the measurement condition may be a condition in which both fingers of the user are in contact with a first electrode and a second electrode of the measurement device and a foot of the user is in contact with a third electrode, so a potential difference between respective electrodes is measured. As another example, when the measurement device is a blood pressure system, the measurement condition may be a condition in which a sensor included in a blood pressure measurement cuff senses an arm of the user.

The activation notification and a measurement start notification may be generated in various forms including a visual method, an auditory method, etc., and two or more methods may be simultaneously used. For example, the activation notification and the measurement start notification may be a scheme in which an LED included in the measurement device is turned on, may be a scheme in which a display included in the measurement device displays an image or text information, and may be a scheme in which a speaker included in the measurement device generates a specific voice signal.

In step S230, the processor 110 included in the measurement device may transmit measurement information to the external device.

According to one embodiment of the present disclosure, the processor 110 included in the measurement device may terminate a measurement operation based on a signal received from the external device. At this time, the signal received from the external device may include a measurement stop signal or a measurement completion signal. Further, the measurement stop signal or measurement completion signal received from the external device may be generate without a direct user input (i.e., a direct manipulation for the external device).

The measurement stop signal may be feedback information transmitted from the external device to the measurement device when an unstable measurement state is identified according to a result of analyzing the measurement information by the external device. At this time, the unstable measurement state may be identified based on at least one of an intensity of the measurement information, an input time interval of the measurement information, and a length of the measurement information. For example, when it is determined that a condition to analyze the measurement information is not satisfied, such as a case where the intensity of the measurement information is weak, a case where the input time interval of the measurement information is long, or a case where the length of the measurement information is short, this may be identified as the unstable measurement state. Meanwhile, the measurement completion signal may be feedback information transmitted from the external device to the measurement device when a measurement completion condition is achieved according to the result of analyzing the measurement information by the external device.

When the measurement device receives the measurement stop signal, the processor 110 included in the measurement device may perform an operation of terminating the measurement operation. In this case, measurement is not normally made, so the operation of deactivating the measurement device may not be included, and it is possible to wait for a next input by maintaining an activation state.

When the measurement device receives the measurement completion signal, the processor 110 included in the measurement device may perform at least one operation of an operation of terminating the measurement operation, an operation of transmitting a standby signal to the external device, and an operation of deactivating the measurement device. When the measurement information is normally transmitted, the measurement and the external device may not be used until next measurement, so the processor 110 terminates the measurement operation or deactivates the measurement device to save power used for driving the measurement device.

Further, in the present disclosure, the measurement end operation may be performed without depending on the signal received from the external device. For example, the measurement device may autonomously perform the measurement end operation after a predetermined time elapsed. At this time, the predetermined time may be different according to the type of measurement device, and a length of the predetermined time may vary according to a setting of the user.

Step S230 may be executed while a measurement program for the measurement device is not executed in a foreground of the external device. That is, the measurement program may be executed in a background state. A foreground process may mean a process which requires a start input or an interaction of the user, or which may be recognized or perceived in real time by the user. Therefore, in the present disclosure, even though there is no input of the user into the external device, the external device may receive the measurement information from the measurement device, and likewise, the external device may generate a measurement stop signal and a measurement completion signal based on an analysis of the measurement information without the input of the user, and transmit the generated signals to the measurement device.

In the present disclosure, the measurement program which is executed in the background state of the external device may be switched to a foreground state when the analysis of the measurement information is completed. At this time, the measurement program may generate a separate notification for notifying, to the user, that the analysis of the measurement information is completed, and then may be switched to the foreground state by the input of the user, or may be automatically switched to the foreground state when the analysis of the measurement information is completed without generating the separate notification. Then, the processor 110 included in the external device may output an analysis result included in the measurement information analysis. At this time, the processor 110 may display an analysis result in the display included in the external device as an example of an output.

According to the present disclosure, the biometric information may be measured without the input of the user into the external device. As a result, by dramatically reducing a required time and hassle for device manipulation upon measuring the biometric information, there is an effect of better response to emergency situations and promoting user convenience.

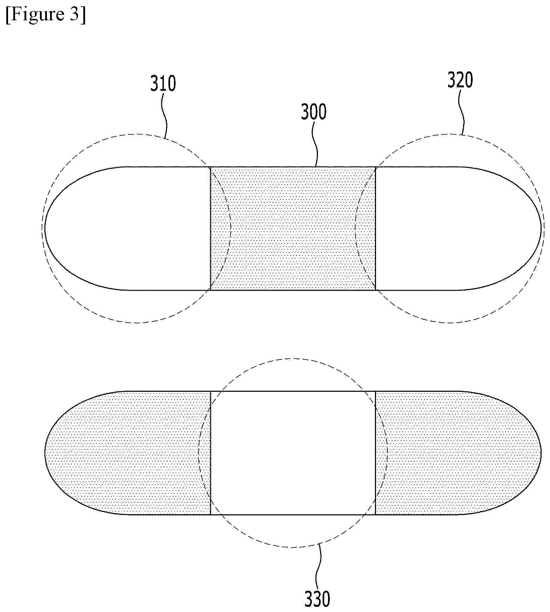

FIG. 3 is an exemplary diagram illustrating a 6-lead hand-held electrocardiogram measurement device among measurement devices according to one embodiment of the present disclosure.

The 6-lead portable electrocardiogram measurement device according to one embodiment of the present disclosure may include a main measurement unit 300. The main measurement unit 300 may include a first electrode 310, a second electrode 320, and a third electrode 330. The first electrode 310, the second electrode 320, and the third electrode 330 may be distinguished according to locations thereof. The first electrode 310, the second electrode 320, and the third electrode 330 may be distinguished according to a target body part of the user to be in close contact. Each electrode may constitute a part of a housing of the main measurement unit. When each electrode constitutes a part of the housing of the main measurement unit, the user may simultaneously possess or use three electrodes without a separate cable. As illustrated in FIG. 3, the main measurement unit 300 may have a pad shape as one embodiment. When the main measurement unit 300 has the pad shape, the first electrode 310 and the second electrode 320 are positioned on both sides of a front portion of the pad, and the third electrode is positioned on a rear surface of the pad, the user may measure an electrocardiogram by contacting the first electrode 310 and the second electrode 320 with thumbs of both hands, respectively and contacting a rear electrode with a part of a foot. An example of the shape of the main measurement unit described above is just an example, and does not limit the present disclosure.

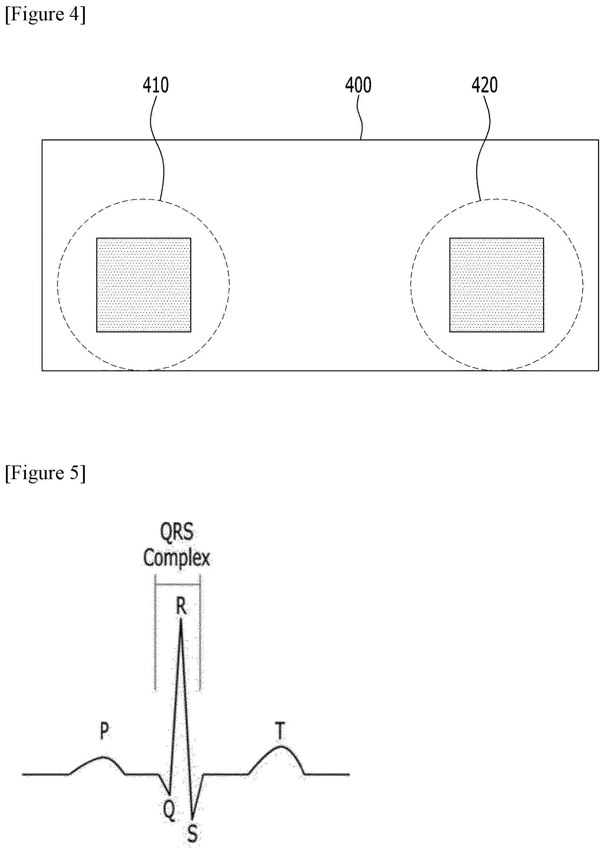

FIG. 4 is an exemplary diagram illustrating a 2-lead hand-held electrocardiogram measurement device among the measurement devices according to one embodiment of the present disclosure.

A main measurement unit 400 of the 2-lead hand-held electrocardiogram measurement device may include a first electrode 410 and a second electrode 420. The first electrode 410 and the second electrode 420 may be distinguished according to locations thereof. The first electrode 410 and the second electrode 420 may be distinguished according to a target body part of a user to be in close contact. Each electrode may configure a part of a housing of the main measurement unit. When each electrode constitutes a part of the housing of the main measurement unit, the user may simultaneously possess or use three electrodes without a separate cable. As illustrated in FIG. 4, the main measurement unit 400 may have a pad shape as one embodiment. When the main measurement unit 400 has the pad shape, the first electrode 410 and the second electrode 420 are positioned on both sides of a front portion of the pad, the user may measure an electrocardiogram by contacting the first electrode 410 and the second electrode 420 with thumbs of both hands, respectively. The example of the shape of the main measurement unit described above is just an example, and does not limit the present disclosure.

In the 6-lead hand-held electrocardiogram measurement device and the 2-lead hand-held electrocardiogram measurement device according to one embodiment of the present disclosure, the main measurement unit 300 or 400 may each include a network unit for wireless data communication. The main measurement unit interacts with an external device via a wired or wireless connection to transmit and receive data. The data communication by the network unit may be performed by a short-range wireless communication method. The short-range wireless communication method may include, for example, a wireless LAN (WLAN), a Bluetooth method, and the like.

The hand-held electrocardiogram measurement device according to one embodiment of the present disclosure may further include an output unit. The output unit may output at least one of information related to an electrical signal measurement of each electrode, information related to an electrocardiogram measurement scheme of a processor included in the hand-held electrocardiogram measurement device, and notification information. The output unit may include at least one of a component for voice output and a component for outputting a video or text. The output unit may be included in an external device linked with the hand-held electrocardiogram measurement device. For example, the output unit may be included in a PC, a smartphone, a tablet terminal, etc., of the user.

A program for the hand-held electrocardiogram measurement device according to one embodiment of the present disclosure may be executed in a background of the external device. The external device may receive electrocardiogram information from the hand-held electrocardiogram measurement device without a separate user input, and analyze the received electrocardiogram information and display the analyzed electrocardiogram information to the user.

FIG. 5 is an exemplary graph showing electrocardiogram measured based on an electrical signal received from an electrode. As illustrated in FIG. 5, a waveform of the electrocardiogram may be distinguished into a P wave, a Q wave, an R wave, an S wave, and a T wave. First, the P wave as a waveform which appears when an atrium is depolarized progresses from the right to the left crossing the atrium. Accordingly, a front portion of the P wave represents depolarization of a right atrium and a rear portion of the P wave represents depolarization of a left atrium.

A QRS complex including the Q wave, the R wave, and the S wave is generated by depolarization of a ventricle. The Q wave represents depolarization of an inter-ventricle septum and remaining parts of the QRS complex represent depolarization of a left/right ventricle which simultaneously occurs.

The T wave is caused by repolarization of the ventricle. The T wave occurs at the end of a ventricular shrink period. The repolarization is progressed slower than the depolarization, and is further widened and is lower in amplitude than the QRS complex. As described above, movement of an internal ventricle and the internal atrium of the heart may be known through the electrocardiogram measured based on the electrical signal received from the electrode.

The electrocardiogram lead included in electrocardiogram data is constituted by a total of 12 leads. The electrocardiogram lead may be divided into a limb lead and a precordial lead. The limb lead may be again divided into standard limb leads including lead I, lead II, and lead III, and augmented limb leads including lead aVR, lead aVL, and aVF. The standard limb lead may be called the same meaning as the standard lead. The augmented limb lead may be called the same meaning as the limb lead. The standard limb lead including three leads among 12 leads is a bipolar lead recording a potential difference of two different electrodes. The remaining leads other than the standard limb lead among 12 leads are a unipolar lead measured based on one electrode.

Throughout this specification, inducement and lead may be interchanged and used, and all of the induction and the inducement and the lead refer to the lead included in the electrocardiogram data.

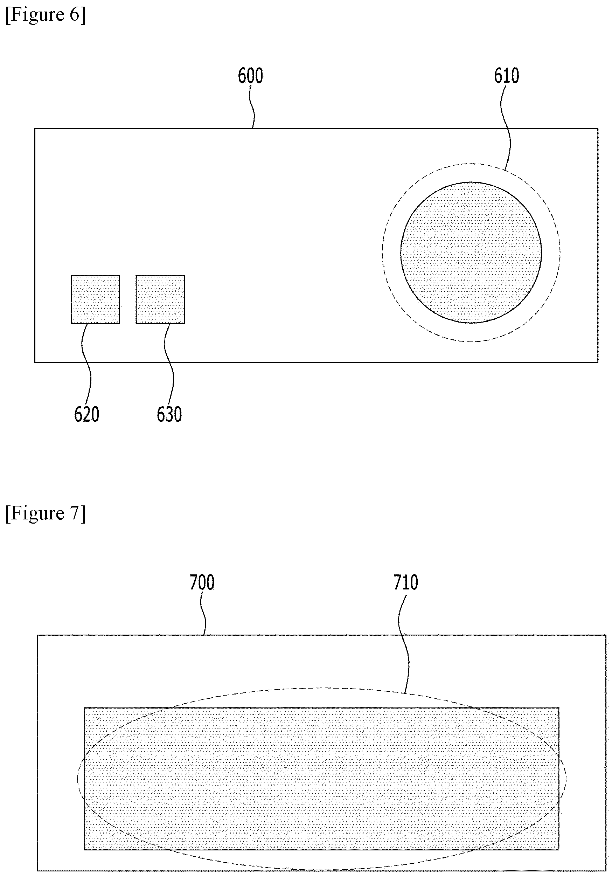

FIG. 6 is an exemplary diagram illustrating a blood pressure measurement device among the measurement devices according to one embodiment of the present disclosure.

The blood pressure measurement device 600 may include a blood pressure measurement cuff 610, a blood pressure measurement start button 620, and a blood pressure measurement stop button 630. By an input of contacting the blood pressure measurement start button or operating the blood pressure measurement device separately, a processor of the blood pressure measurement device may identify a measurement attempt applied to the blood pressure measurement device.

When a sensor included in the blood pressure measurement cuff 610 identifies that an arm of a user is entered into the blood pressure measurement cuff, the processor of the blood pressure measurement device may start a blood pressure measurement. When the arm of the user moves out of a predetermined range or an external force of a predetermined level or more is applied to the blood pressure measurement device during the measurement, or when a stop signal is received from the external device linked with the blood pressure measurement device is received, the processor of the blood pressure measurement device may perform all operations of stopping the measurement attempt, deactivating the measurement device, and transmitting an idle signal to the external device.

The blood pressure measurement device according to one embodiment of the present disclosure may further include an output unit. The output unit may output at least one of information on a measured blood pressure of the user, and measurement state information notification information. The output unit may include at least one of a component for voice output and a component for outputting a video or text. The output unit may be included in an external device linked with the blood pressure measurement device. For example, the output unit may be included in a PC, a smartphone, a tablet terminal, etc., of the user.

A program for the blood pressure measurement device according to one embodiment of the present disclosure may be executed in a background of the external device. The external device may receive the information on the blood pressure of the user electrocardiogram information from the blood pressure measurement device without a separate user input, and analyze the received information and display the analyzed information to the user.

FIG. 7 is an exemplary diagram illustrating a weight scale among the measurement devices according to one embodiment of the present disclosure.

The weight scale 700 may include a pressure detection unit 710. By an input of applying a pressure to the pressure detection unit 710, or operating the weight scale separately, a processor of the weight scale may identify a measurement attempt applied to a blood pressure weight scale.

When a sensor included in the pressure detection unit 710 identifies that a pressure applied to the sensor is equal to or higher than a predetermined level, the processor of the weight scale may start a weight measurement. When a pressure sensed by a pressure sensor is rapidly changed during the measurement or when the stop signal is received from the external device linked with the weight scale, the processor of the weight scale may perform all operations of stopping the measurement attempt, deactivating the measurement device, and transmitting the idle signal to the external device.

The weight scale according to one embodiment of the present disclosure may further include an output unit. The output unit may output at least one of information on a measured weight of the user, and measurement state information notification information. The output unit may include at least one of a component for voice output and a component for outputting a video or text. The output unit may be included in an external device linked with the weight scale. For example, the output unit may be included in a PC, a smartphone, a tablet terminal, etc., of the user.

A program for the weight scale according to one embodiment of the present disclosure may be executed in a background of the external device. The external device may receive the information on the weight of the user from the weight scale without a separate user input, and analyze the received information and display the analyzed information to the user.

As illustrated in FIGS. 3, 4, 6, and 7, the measurement device according to the present disclosure may be a device that measures biometric information including an electrocardiogram, a blood pressure, and a weight. However, the measurement device according to the present disclosure is not limited to the measurement devices presented in the drawings, but the method of the present disclosure may be used in measurement equipments for measuring other biometric information such as electromyogram, electroencephalogram, etc.

In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.

Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a learning rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

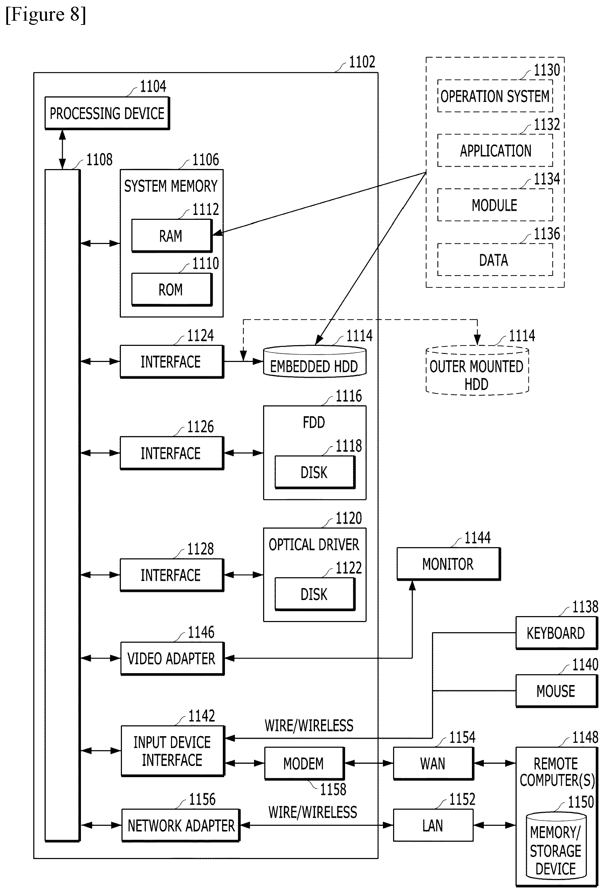

FIG. 8 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

**In** general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

### [Best Mode]

Related contents in the best mode for carrying out the present disclosure are described as above.

## Claims

1. A method performed by a measurement device for measuring biometric information, the method comprising:
identifying a measurement attempt applied to the measurement device;
performing both an operation of activating the measurement device and an operation of transmitting a wake-up signal to an external device, in response to the identified measurement attempt; and
transmitting measurement information to the external device.

2. The method of claim 1, wherein the transmitting of the measurement information to the external device is performed while a measurement program for the measurement device is not performed in a foreground of the external device.

3. The method of claim 1, wherein the performing of both the operation of activating the measurement device and the operation of transmitting the wake-up signal to the external device, in response to the identified measurement attempt further includes:
generating an activation notification, and
generating a measurement start notification based on identifying whether the measurement attempt meets a measurement start condition.

4. The method of claim 1, further comprising:
performing at least one of an operation of deactivating the measurement device or an operation of transmitting an idle signal to the external device when the identified measurement attempt is stopped.

5. The method of claim 1, further comprising:
performing an operation of deactivating the measurement device.

6. The method of claim 1, further comprising:
terminating a measurement operation based on a signal received from the external device,
wherein the terminating of the measurement operation based on the signal received from the external device includes:
terminating the measurement operation when the signal is a measurement stop signal,
performing at least one of an operation of terminating the measurement operation, an operation of transmitting a standby signal to the external device, or an operation of deactivating the measurement device when the signal is a measurement completion signal, or
terminating the measurement operation when a predetermined time elapsed from a time when the signal is received.

7. A method performed by a computing device, the computing device operating by linking with a measurement device, the method comprising:
executing a program for the measurement device in a background when a wake-up signal is received from the measurement device; and
analyzing measurement information received from the measurement device based on the program executed in the background.

8. The method of claim 7, further comprising:
transmitting, when an unstable measurement state is identified through the analysis, feedback information for the unstable measurement state to the measurement device.

9. The method of claim 8, further comprising:
outputting an analysis result by switching the program into a foreground state when the analysis of the measurement information is completed.

10. The method of claim 8, wherein the unstable measurement state is identified based on at least one of an intensity of the measurement information, an input time interval of the measurement information, or a length of the measurement information.

11. A computer program stored in a computer readable storage medium, the computer program including instructions allowing a measurement device to perform operations of measuring biometric information, the operations comprising:
an operation of identifying a measurement attempt applied to the measurement device;
an operation of activating the measurement device and transmitting a wake-up signal to an external device, in response to the identified measurement attempt; and
an operation of transmitting measurement information to the external device.

12. A measurement device for obtaining biometric information, comprising:
at least one processor;
a measurement unit for receiving biometric information; and
a communication unit for communicating with an external device,
wherein the at least one processor is configured to:
identify a measurement attempt applied to the measurement unit,
perform both an operation of activating the measurement device and an operation of transmitting a wake-up signal to an external device, in response to the identified measurement attempt, and
transmit measurement information to the external device.
